# EUROPEAN PATENT SPECIFICATION

(11) **EP 3 611 183 B1**
(45) Date of publication and mention of the grant of the patent: **27.12.2023**
(21) Application number: 18784269.5
(22) Date of filing: 10.04.2018
(51) Int. Cl.: C07K 7/06, G01N 33/543, G01N 33/564, C07K 14/47

(54) **EPITOPE PEPTIDE SPECIFICALLY RECOGNIZING AUTOANTIBODY AGAINST AQUAPORIN 5, AND USE THEREOF**
EPITOP-PEPTID MIT SPEZIFISCH ERKENNUNG VON AUTOANTIKÖRPER GEGEN AQUAPORIN 5 UND DESSEN VERWENDUNG
ÉPITOPE PEPTIDIQUE RECONNAISSANT SPÉCIFIQUEMENT UN AUTO-ANTICORPS DIRIGÉ CONTRE L'AQUAPORINE 5, ET SON UTILISATION

(30) Priority: 12.04.2017 KR 20170047244
(43) Date of publication of application: 19.02.2020
(73) Proprietor: Seoul National University R & DB Foundation, Seoul 08826 (KR)
(72) Inventor: CHOI, Youngnim, Seoul 03174 (KR)
(74) Representative: Ter Meer Steinmeister & Partner
(86) International application number: PCT/KR2018/004190
(87) International publication number: WO 2018/190612

(56) References cited:
- WO-A1-2013/057599
- WO-A1-2016/175406
- CN-A- 103 275 987
- JP-A- 2008 249 332
- JP-B2- 3 504 524
- KR-A- 20160 128 647
- US-A1- 2016 000 794
- N.-Y. KOO ET AL: "Functional epitope of muscarinic type 3 receptor which interacts with autoantibodies from Sjogren's syndrome patients", RHEUMATOLOGY, vol. 47, no. 6, 11 March 2008 (2008-03-11) , pages 828-833, XP055753250, GB ISSN: 1462-0324, DOI: 10.1093/rheumatology/ken064
- V GRESZ ET AL: "Immunolocalization of AQP5 in resting and stimulated normal labial glands and in Sj?gren's syndrome", ORAL DISEASES, vol. 21, no. 1, 21 April 2014 (2014-04-21) , pages e114-e120, XP055326237, GB ISSN: 1354-523X, DOI: 10.1111/odi.12239
- J. ALAM ET AL: "Functional Epitopes for Anti-Aquaporin 5 Antibodies in Sjögren Syndrome", JOURNAL OF DENTAL RESEARCH, vol. 96, no. 12, 30 June 2017 (2017-06-30) , pages 1414-1421, XP055559408, US ISSN: 0022-0345, DOI: 10.1177/0022034517717965
- ALAM, J.: "Detection of autoantibodies against aquaporin-5 in the sera of patients with primary Sjogren' s syndrome", Immunol. Res., vol. 64, no. 4 August 2016 (2016-08), pages 848-856, XP036000249, Retrieved from the Internet: URL:doi: 10.1007/s12026-016-8786-x
- SOVERAL, G. et al.: "Aquaporin modulators: a patent review (2010-2015)", Expert Opinion on Therapeutic Patents, vol. 27, no. 1 26 September 2016 (2016-09-26), pages 49-62, XP055559405, Retrieved from the Internet: URL:DOI: 10.1080/13543776.2017.1236085
- ALAM, J.: "Functional epitopes for anti-aquaporin 5 antibodies in sjögren syndrome", Journal of Dental Research, vol. 96, no. 12 30 June 2017 (2017-06-30), pages 1414-1421, XP055559408, Retrieved from the Internet: URL:doi: 10.1177/0022034517717965

## Description

### BACKGROUND OF THE INVENTION

### Field of the Invention

The present application relates to the epitopes specifically recognizing AQP5 autoantibody found in patients with Sjogren's syndrome and their detection and therapeutic use for Sjogren's syndrome.

### Description of the Related Art

Sjogren's syndrome is a disease characterized mainly by dryness of the mouth and eyes and by dysfunction of the salivary and lacrimal glands by autoimmune responses. Criteria for diagnosing Sjogren's syndrome are generally based on the result of 1) salivary gland scan to examine dryness of the mouth and eyes, Schirmer test, 2) anti-Ro, anti-La autoantibody test, or 3) biopsy to examine the lymphocyte deposition in the salivary glands.

However, such criteria do not provide diagnostic factors in relation to the disease activity and that can explain the cause of salivary gland and lacrimal gland dysfunction.

US Patent application publication NO. 2010/0136532A1 relates to polymorphism of AQP5 and discloses a method of predicting or diagnosing cancer based on the polymorphism found in AQP5. There is no disclosure in relation to diagnosis or treatment of Sjogren's syndrome.

Korean patent application publication NO. 2016-0128647 relates to a method of diagnosing Sjogren's syndrome based on the antibody test by detecting the presence of anti-AQP5 autoantibody. This application is based on the findings of the mechanism that anti-AQP5 autoantibody binds to AQP5 and prevents water molecules from moving through the cell membrane thus leading to the reduction of saliva and tear secretion and discloses the use of anti-AQP5 autoantibody as a diagnostic marker for Sjogren's syndrome.

However, the above application is limited in that by using a full-length AQP5 as an antigen, all kinds of anti-AQP5 autoantibodies present in the body regardless of whether they affect the function of AQP5 or not are detected thus leading to a very low specificity and even 32% of the healthy subject showed positive results for the autoantibodies. Thus, there is a need to develop epitopes and diagnostic method with high specificity.
WO2016175406 A1 discloses a method for diagnosing Sjogren's syndrome based on detecting AQP5 autoantibody.
WO2013057599 A1 discloses the diagnoses or prognosis of an autoimmune disease and cancer in a subject involving detecting autoantibodies.
N.-Y. KOO ET AL, "Functional epitope of muscarinic type 3 receptor which interacts with autoantibodies from Sjogren's syndrome patients", RHEUMATOLOGY, GB, (20080311), vol. 47, no. 6, pages 828 - 833, discloses the determination of a functional epitope of muscarinic type 3 (M3R) receptor.

V GRESZ ET AL, "Immunolocalization of AQP5 in resting and stimulated normal labial glands and in Sjögren's syndrome", ORAL DISEASES, GB, (20140421), vol. 21, no. 1, pages e114 - e120, determines the effect of oral pilocarpine on the localization of AQP5 in human labial minor salivary glands.

### SUMMARY OF THE INVENTION

The present disclosure is to provide epitopes to specifically detect or recognize aquaporin 5 autoantibodies, particularly ones that affect the function of aquaporin 5 and uses thereof.

In one aspect of the present disclosure, there is provided a polypeptide having an amino acid sequence selected from the group consisting of GCSMNPARSFGC (SEQ ID NO: 5), CMNPARSFGC (SEQ ID NO: 6), CWPSALPTC (SEQ ID NO: 2); and CNNNTTQGC (SEQ ID NO: 3).

In the foregoing peptides or epitopes, they may form a circular structure and functions as a circular structure.

In the foregoing peptides or epitopes, the polypeptide or epitopes with SEQ ID NO: 2, SEQ ID NO: 3, SEQ ID NO: 5, and SEQ ID NO: 6 may form a circular structure by binding between the cysteine residues included in each sequence.

In another aspect of the present disclosure, there is provided a nucleotide encoding the foregoing polypeptides.

In other aspect of the present disclosure, there is provided a vector comprising the foregoing nucleotides.

In other aspect of the present disclosure, there is provided a cell comprising the foregoing vector or the nucleotide, wherein the cell includes a prokaryote or a eukaryote.

In other aspect of the present disclosure, there is provided a composition for detecting AQP5 autoantibodies comprising at least one polypeptide or epitope as disclosed herein.

In other aspect of the present disclosure, there is provided a pharmaceutical composition for treating Sjogren's syndrome comprising at least one polypeptide or epitope as disclosed herein.

In a reference aspect, there is provided a method of diagnosing Sjogren's syndrome using the polypeptide or the epitope as disclosed herein.

In other aspect of the present disclosure, there is provided a method of detecting AOP5 autoantibody using the polypeptide or the epitope as disclosed herein.

In the foregoing methods of detecting AOP5 autoantibody, the method may comprise the steps of: providing at least one of the polypeptide or epitope of the present disclosure; providing a biological sample that has been obtained from a subject in need of detection of AQP5 autoantibody; and contacting the sample with the polypeptide or epitope of the present disclosure to detect a binding of AQP5 autoantibody to the polypeptide and correlates or the detection results with the presence of AQP5 autoantibody in the sample.

In the foregoing method, the method may be performed on at least one sample including a whole blood, a serum, a plasma, a tear or a saliva.

In the foregoing method, wherein the detection result positive for binding between the epitope and the autoantibody compared to a control indicates the sample or the subject from which the sample is derived is inflicted with Sjogren's syndrome.

In other reference aspect, there is provided a method of diagnosing Sjogren's syndrome by detecting the AQP5 autoantibody using the polypeptide or the epitope as disclosed herein.

In the foregoing methods of diagnosis, the method comprises the steps of: providing a sample from a subject in need of detection of AQP5 autoantibody or diagnosis of Sjogren's syndrome; contacting the sample with the polypeptide or the epitope of the present disclosure to detect a binding between AQP5 autoantibody and the polypeptide, wherein the binding indicates the presence of the autoantibody and the subject is inflicted with Sjogren's syndrome.

In the foregoing methods, the method may be performed with at least one sample including a whole blood, a serum, a plasma, a tear or a saliva.

In the foregoing methods, the detection of the binding between the epitope or the polypeptide of the present disclosure and the autoantibody may be performed by FACS (Fluorescence-activated cell sorting), Cell immunostaining, Radial Immunodiffusion, Reverse Current Electrophoresis including Immunoelectrophoresis, or Immunoprecipitation, RIA (Radioimmunoassay) or ELISA (Enzyme Linked Immunosorbent Assay).

In the foregoing methods, the binding between the polypeptide or the epitope and the autoantibody results in the formation of a complex and the detection step includes a use of a detection antibody which specifically recognizes the autoantibody in the complex, wherein the detection antibody specifically recognizes a human IgG or IgA and is labelled with chromophores including an enzyme such as alkaline phosphatase, biotin, beta-galactosidase or peroxidase; radioactive material; or a material including color particles such as colloidal gold particles or color latex particles.

In one embodiment of the foregoing methods, the polypeptide or the epitope of the present disclosure is labelled with a biotin, in which case, the step of detecting the binding between the epitope and the autoantibody includes contacting the biotin labelled epitope with a solid substrate labelled with or onto which avidin or streptavidin are attached; applying the sample to the solid substrate to allow a binding between the autoantibody in the sample and the polypeptide attached to the solid substrate via biotin-avidin, or biotin-streptavidin complex formation; and apply a detection antibody that specifically detects the autoantibody bound to the polypeptide, wherein the detection antibody specifically recognize a human IgG or IgA and may be labelled with chromophores including an enzyme such as alkaline phosphatase, biotin, beta-galactosidase or peroxidase; radioactive material; or a material including color particles such as colloidal gold particles or color latex particles.

In another reference aspect of, there is provided a kit for detecting or diagnosing Sjogren's syndrome in a sample comprising at least one polypeptide of the present disclosure and a detection antibody, wherein the detection antibody specifically recognizes a human IgG or IgA and is labelled with a chromophore including an enzyme such as alkaline phosphatase, biotin, beta-galactosidase or peroxidase; radioactive material; or a material including color particles such as colloidal gold particles or color latex particles.

In the foregoing kits, it further comprises a solid substrate onto which streptavidin or avidins are attached, and the polypeptide is labelled with a biotin at its Nor C- terminal.

In the foregoing kits, the present polypeptide or the epitope may be provided as bound to a solid substrate such as a microplate, microarray, chip, glass, bead or particle, or membrane.

In other aspect of the present disclosure, there is provided a use of the present polypeptide or epitope for detecting AQP5 autoantibody, wherein the peptide has an amino acid sequence selected from the group consisting of GCSMNPARSFGC (SEQ ID NO: 5), CMNPARSFGC (SEQ ID NO: 6), CWPSALPTC (SEQ ID NO: 2); and CNNNTTQGC (SEQ ID NO: 3).

### ADVANTAGEOUS EFFECTS

The epitopes disclosed herein can be used to detect anti-aquaporin 5 autoantibodies that inhibit the function aquaporin 5 in the body, and the epitopes according to the present invention can also eliminate/neutralize the activity of anti-aquaporin 5 autoantibodies, resulting in reversing the inhibitory effect on tear/saliva secretion by the autoantibodies. Thus, the present polypeptides can be also effectively used as a therapeutic agent. In addition, the specificity of detecting anti-aquaporin 5 autoantibody has been greatly improved by the present epitopes compared with using the whole AQP5 protein as an antigen. In particular, the normal control group was detected positive for aquaporin 5 autoantibody in about 30% by using the AQP5 protein as an antigen, but it was reduced to about 5% using the epitope according to the present application.

### BRIEF DESCRIPTION OF THE DRAWINGS

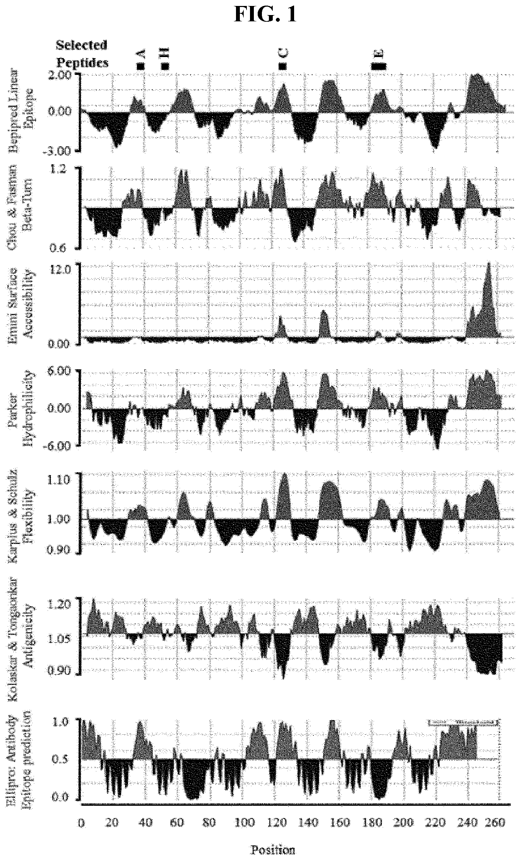

Fig. 1 is a diagram showing the selection of peptides in human AQP5 sequence based on the prediction results of B cell epitopes. Each diagram presents the results of B-cell linear epitope prediction of human AQP5 using the indicated *in silico* tool. Grey areas above the y-axis threshold represent the regions that are likely part of B cell epitopes, while the x-axis represents the position of amino acid sequence. The locations of selected peptides along the amino acid sequence are shown on top.

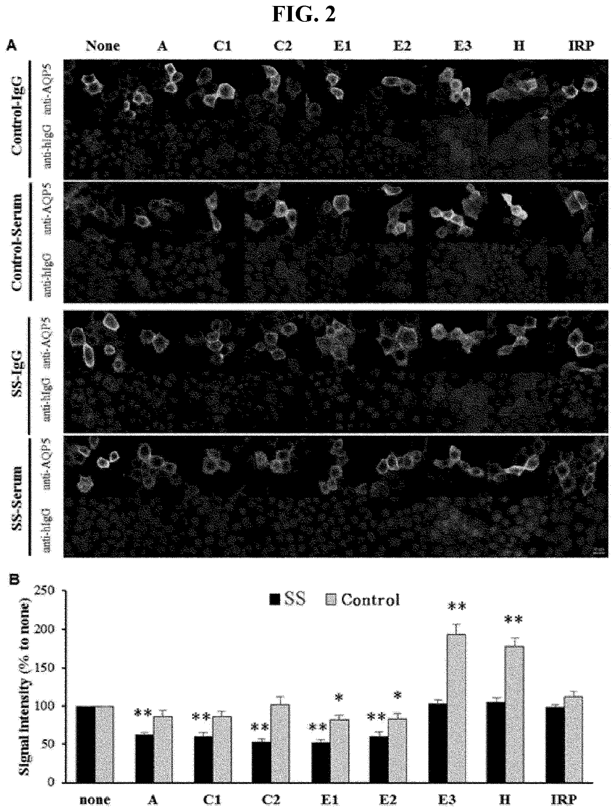

Fig. 2 shows the result of epitope mapping of anti-AQP5 autoantibodies detected in control and SS samples by Indirect Immunofluorescence assay. A mixture of MDCK and MDCK-AQP5 cells was double stained with goat anti-AQP5 antibodies and either human serum or purified IgG that was pre-incubated with an indicated peptide, followed by Alexa Fluor 488-conjugated anti-goat IgG (green) and CFTM 594-conjugated anti-human IgG (red). (A) Representative results by control-IgG, control serum, SS-IgG, and SS-serum are shown. (B) The intensities of the red signals co-localized with green were determined and expressed as the percentages of those obtained in the absence of peptides. The means and standard errors of 27 SS and 14 control samples presented in Table 2 are shown. *, *p* < 0.05; ***, p <* 0.0001 compared to none. The figure shows that epitopes according to the present disclosure can preferentially detect AQP5 autoantibodies in the patients with Sjogren's syndrome in rather than those present in the control healthy subject.

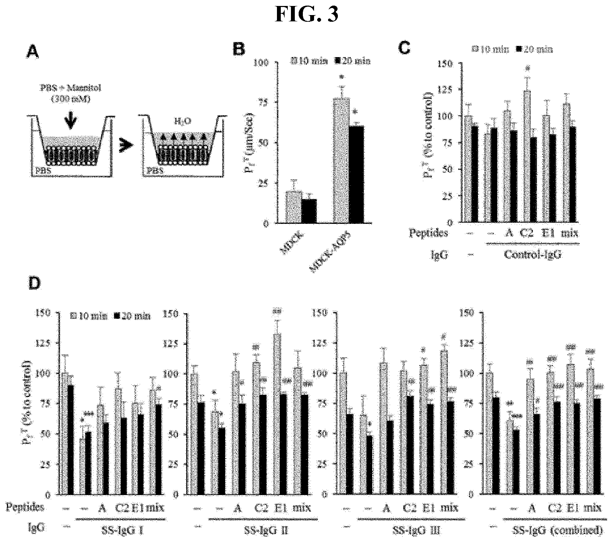

Fig. 3 shows the functional epitopes on AQP5 found in the present disclosure by measurements of transepithelial osmotic water flow. (A) Schematic presentation of osmotically induced transepithelial water movement across MDCK-AQP5 cells grown on porous supports. Water transport from the basal to apical side increases the volume of the apical solution. (B) Transepithelial water permeability (P_{f}^{T}) in MDCK/MDCK-AQP5 cells were determined 10 and 20 minutes after osmotic challenge. *, *p* < 0.0001 compared to MDCK. (C-D) The effects of control- or SS-IgG and neutralizing peptides on P_{f}^{T} in MDCK-AQP5 cells were determined 10 and 20 minutes after osmotic challenge. Data are expressed as percentage of the P_{f}^{T} in MDCK-AQP5 cells alone at 10 minutes. *, *p* < 0.05; **, *p* < 0.01; ***, *p* < 0.001 compared to MDCK-AQP5 cells alone. #, *p* < 0.05; *##, p* < 0.01; ###, *p* < 0.001 compared to IgG alone without peptides. The figure shows the recovery of AQP5 function by the epitope peptides of the present disclosure in the presence of anti-AQP5 autoantibodies, indicating that the epitope peptides according to the present invention can be advantageously used as a therapeutic agent for treating Sjogren's syndrome.

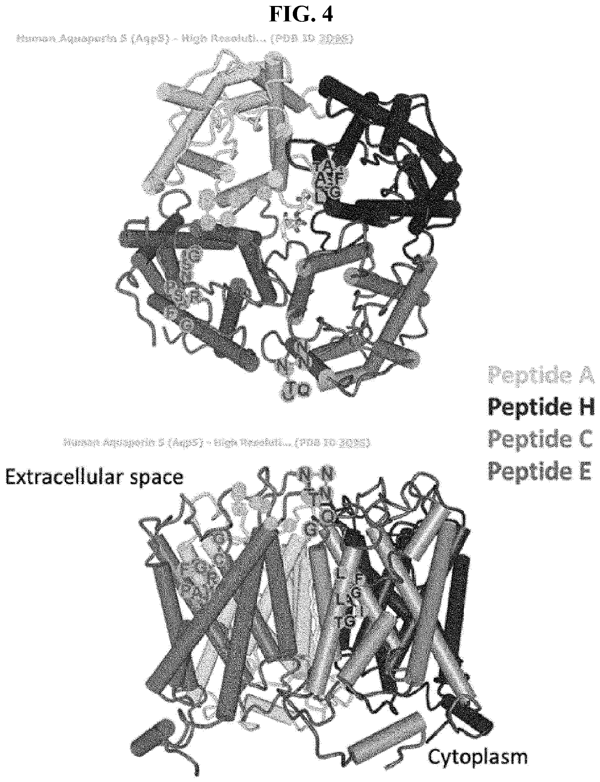

Fig. 4 shows the locations of selected peptides in the 3D structure of AQP5. In an X-ray structure of the human AQP5 tetramer, sequences of selected peptides are highlighted and schematically depicted using the iCn3D software. Although the selected peptides are located in all four protomers of the AQP5 tetramer, the location of each peptide is highlighted in the different protomers to visualize each of the four peptides in different colors. (Top panel) Top view of the tetramer. (Bottom panel) Side view of the tetramer. This figure supports the importance of the circular structure in peptide E of the epitope peptides according to the present application.

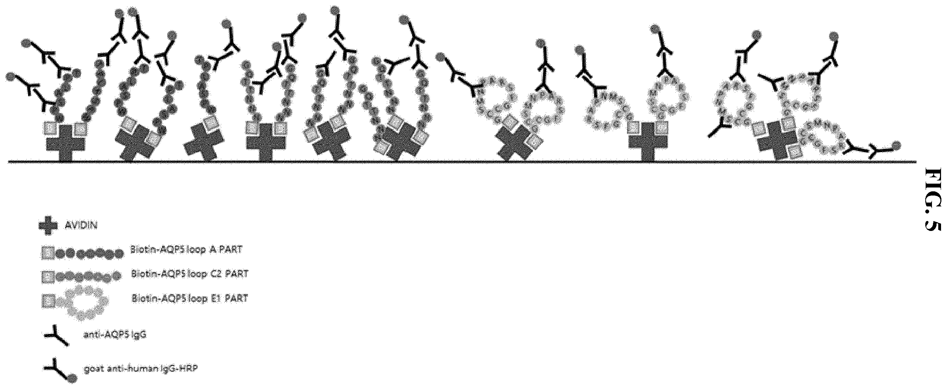

Fig. 5 is a schematic diagram showing one example of a strategy for detecting anti-AQP5 autoantibody present in a sample using the epitope according to the present disclosure by ELISA.

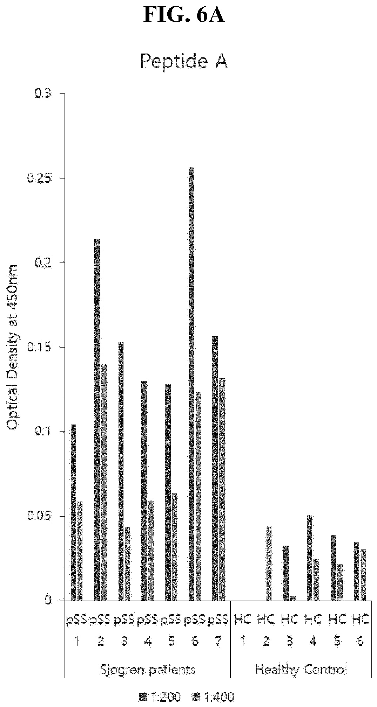

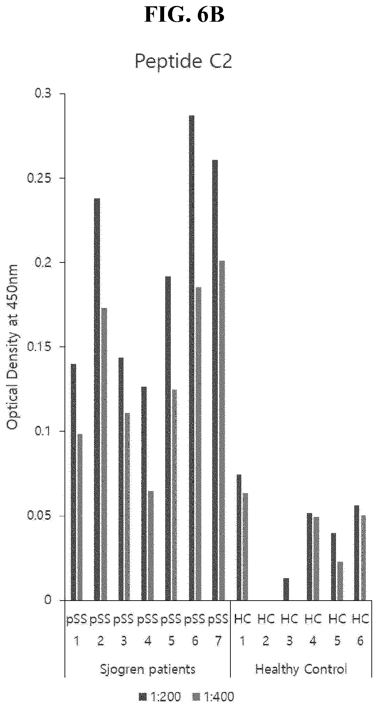

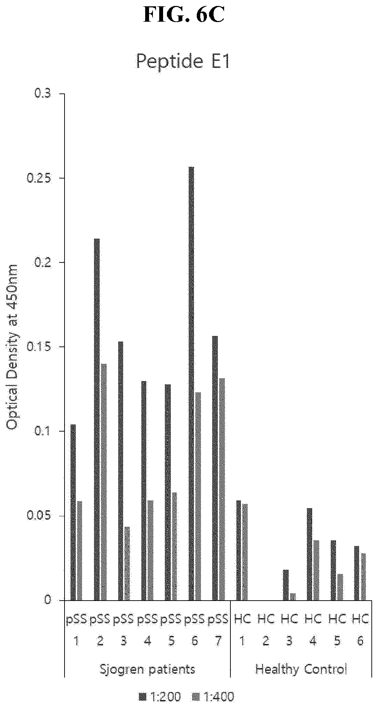

Fig. 6A, 6B and 6C show experimental results performed according to the strategy as in Fig. 5. The results indicate that the epitope according to the present application can be applied to an ELISA to effectively detect the autoantibodies present in the sample simply and effectively.

### DETAILED DESCRIPTION OF THE EMBODIMENTS

The present disclosure is based, in part, on the discovery and characterization of novel epitopes found in protein aquaporin 5 (AQP5) that can specifically recognize or bind to AQP5 autoantibodies. In particular, it was found in the present disclosure that the epitopes located in the three extracellular loops of the AQP5 protein and the second helix region of AQP5 protein specifically bind to AQP5 autoantibodies. It has also been found herein that not only the linear epitopes but also the epitopes with circular structure can effectively act on the binding of AQP5 autoantibodies. In addition, the present peptides or epitope can be able to detect the AQP5 autoantibodies that can affect the function aquaporin 5 in cells among various AQP5 autoantibodies generated in cells. Thus, the present peptides or epitopes can be advantageously used for treating Sjogren's syndrome by reversing the inhibitory effect onAQP5 function by the autoantibodies.

Thus, in one aspect, the present disclosure relates to polypeptides or epitopes of AQP5, wherein the polypeptide is represented by the amino acid sequence: GCSMNPARSFGC(SEQ ID NO: 5), or CMNPARSFGC(SEQ ID NO: 6).

The term "epitope" as used herein refers to an antigen that can be specifically recognized by a particular antibody. In the present disclosure, the epitope is also referred to as a polypeptide. Throughout this application, epitopes and peptides may be used interchangeably. In addition, isolated or purified protein or peptide molecules comprising the epitope of the present invention that are larger than the epitope of the present application are still included in the scope of the present application.

The epitopes according to the invention are derived from AQP5. AQP5 is a water channel protein expressed in acinar cells and duct cells of salivary glands and lacrimal glands, and provides a path through which water molecules pass through lipid membranes. AQP5 consists of six transmembrane alpha helices linked by three extracellular and two intracellular loops as described in FIG. 4. Extracellular loop E and intracellular loop B directly contribute to the channel formation as two asparagine-proline-alanine (NPA) motifs from each loop overlap in the cell membrane.

The epitopes according to the present disclosure are located in specific regions of AQP5. The epitope of SEQ ID NO: 1 is located in the extracellular loop A region corresponding to amino acid sequences 35-41 (based on the sequence of SEQ ID NO: 8) of the human APQ5 protein. The peptide of SEQ ID NO: 4 according to the present application is located in extracellular loop C corresponding to amino acid sequences 123-129 (based on the sequence of SEQ ID NO: 8). The epitopes of SEQ ID NO: 5 and 6 are located in extracellular loop E located at amino acid sequences 181-191 and 184-191 (based on the sequence of SEQ ID NO: 8), respectively.

The present epitope is characterized by the sequences derived from the specific regions as described above, as well as the structure formed by the epitopes. Specifically, it has been found in the present disclosure that epitopes having linear and/or circular structure depending on their location in AQP5 play an important role in acting as an antigen in effectively detecting AQP5 autoantibodies in accordance with the present disclosure.

Thus, in one embodiment of the present invention, the epitope may be used as linear peptides, or cysteine residues are added at the N- and/or C-terminus of the peptides to make structural modifications. Or the present epitope may form a circular structure by themselves without addition of the residues by the bonds formed between the amino acid residues present at both of or near the N- and C-terminus.

In one embodiment of the present disclosure, the epitopes located in the extracellular region A or C loop may exhibit excellent effects of the present disclosure as a linear structure or as a circular structure which may be formed by introducing a cysteine residue(s) having a side chain SH group or a residue equivalent thereof at the N and/or C terminus. The circular structure may be also formed without the addition of extra residues.

In one embodiment, such modifications include the addition of one cysteine residue at each of the N- and C-terminus of SEQ ID NO: 1, which results in the amino acid sequences of SEQ ID NO: 2 (CWPSALPTC), or the addition of one cysteine residue at each of the N- and C-terminus of SEQ ID NO: 4, which results in the amino acid sequences of SEQ ID NO: 3 (CNNNTTQGC). A circular structure may be formed by forming a covalent bond between the added cysteine residues.

The epitopes present in the region E according to the present invention acts as a circular structure, the linear structure was found not to bind to the autoantibody as described below. In one embodiment the epitopes present in the E region are each represented by the amino acid sequence of SEQ ID NO: 5 (GCSMNPARSFGC) or of SEQ ID NO: 6 (CMNPARSFGC). The sequences have been modified to form a circular structure by adding one cysteine residue only at the C terminus of the sequence of SEQ ID NO: 5, in which case a circular structure is formed between the second cysteine residue from the N-terminal and the added cysteine residue, or by adding one cysteine residue at each of the N- and C- terminus of the sequence of SEQ ID NO: 6 in which case a circular structure is formed between the added cysteine residues at each end.

In the present epitopes, the amino acid sequence as well as their conformations are important for the proper function of the epitope according to the present invention.

The polypeptides according to the present invention are not limited to the sequences as disclosed herein but include biological equivalents thereof. Such modifications include, for example, a deletion, insertion and/or substitution of one or more residues in the amino acid sequence. The modifications may be determined in consideration of properties of the similarity of side chains such as sizes, charges, hydrophobic or hydrophilicity. Based on the characteristics of the side chains in terms of size, shape and chemical/electrical properties, it is considered that arginine, lysine and histidine are positively charged residue; alanine, glycine, and serine are having similar size of side chains; phenylalanine, tryptophan and tyrosine are having similar structure of side chains.

Thus, in consideration of this, arginine, lysine and histidine; alanine, glycine and serine; phenylalanine, tryptophan and tyrosine are considered biologically equivalent. Furthermore, when considering variants having biologically equivalent activities as described above, it is encompassed in the present invention not only the amino acid sequences disclosed herein or nucleic acids encoding the same as described below, also the sequences with substantial identity to the sequences disclosed herein. The term "substantial identity" refers to those showing preferably at least 61%, more preferably at least 70%, still more preferably at least 80%, most preferably at least 90% similarity to the sequence disclosed herein, when aligning sequences with the sequence disclosed herein so as to correspond to each other to the highest possible extent and analyzing the aligned sequences using algorithms that are generally used in the art. Methods of alignment of sequences for comparison are well-known in the art. Various programs and alignment algorithms are described in, for example, Smith and Waterman, Adv. Appl. Math. (1981) 2:482; Needleman and Wunsch, J. Mol. Bio. (1970) 48:443; Pearson and Lipman, Methods in Mol. Biol. (1988) 24: 307-31; Higgins and Sharp, Gene (1988) 73:237-44; Higgins and Sharp, CABIOS (1989) 5:151-3; Corpet et al., Nuc. Acids Res. (1988) 16:10881-90; Huang et al., Comp. Appl. BioSci. (1992) 8:155-65 and Pearson et al., Meth. Mol. Biol. (1994) 24:307-31. The NCBI Basic Local Alignment Search Tool (BLAST) (Altschul et al., J. Mol. Biol. (1990) 215:403-10) is available from the NBCI and the like, for use in connection with the sequence analysis programs such as blast, blastp, blasm, blastx, tblastn and tblastx. The BLAST can be accessed at http://www.ncbi.nlm.nih.gov/BLAST/. A description of how to determine sequence identity using this program is available at http://www.ncbi.nlm.nih.gov/BLAST/blast_help.html.

In other aspect, the present disclosure relates to a nucleic acid, a vector comprising the same, and a cell including eukaryotic or prokaryotic cells transformed with the vector.

As used herein, the term "nucleic acid" is meant to encompass DNA (gDNA and cDNA) and RNA molecules inclusively, and the nucleotides, which are the basic building blocks of nucleic acid molecules, include not only natural nucleotides but also analogues thereof with modified sugars or bases (Scheit, Nucleotide Analogs, John Wiley, New York(1980); Uhlman and Peyman, Chemical Reviews, (1990) 90:543-584). The sequences of nucleic acid molecules can be determined from the amino acid sequence from a codon table and can also be modified. Such modifications include addition, deletion, or non-conservative or conservative substitutions of nucleotides. Nucleic acid molecules herein are also construed to include nucleotide sequences that exhibit substantial identity to the nucleotide sequences described above. The substantial identity refers to those showing preferably at least 80%, more preferably at least 90%, still more preferably at least 95% similarity to the sequence disclosed herein, when aligning sequences with the sequence disclosed herein so as to correspond to each other to the highest possible extent and analyzing the aligned sequences using algorithms that are generally used in the art.

The recombinant vectors comprising a nucleic acid according to the present invention may include a plasmid vector; cosmid vector; viral vectors such as bacteriophage vectors, adenovirus vectors, retrovirus vectors, and adeno-associated virus vectors, and the like as a means for expressing a target gene in a host cell. Nucleic acid molecules in such vectors are operably linked to regulatory sequences such as promoters, signal sequences, or arrays of transcriptional regulator binding sites for expression so that they can be regulated and expressed under the control of the regulatory sequences. The recombinant vector system herein can be constructed using various methods known in the art for example refer to: Sambrook et al., Molecular Cloning, A Laboratory Manual, Cold Spring Harbor Laboratory Press (2001).

A transformant, or prokaryotic or eukaryotic cell, comprising a recombinant vector according to the present invention is a cell transformed or transfected with the vector according to the present invention. In the present disclosure "transformation" and/or "transfection" includes any methods that can deliver nucleic acids to cells, tissue or organs and can be performed by standard methods known in the related art depending on the cell types employed. These methods include, but are not limited to, electroporation, protoplast fusion, calcium phosphate (CaPO₄) precipitation, calcium chloride (CaClz) precipitation, agro bacteria mediated transformation, PEG, dextran sulfate, liposomes, and the like. The culture of transformant can be performed using suitable media and culture conditions as known in the art. For example, the culture methods may be found: James M. Lee, Biochemical Engineering, Prentice-Hall International Editions, 138-176. The epitopes obtained by culturing the vector-transformed transformant can be used without purification, or may be further purified to a high purity using various conventional methods, for example, dialysis, salt precipitation and chromatography.

The polypeptides according to the present invention, in order to protect from protein cleavage enzymes and increase stability in vivo, the N-terminal and/or C-terminal, etc. may be chemically modified or protected with an organic functional group, or amino acid residues may be added to the peptide terminus. In particular, in the case of chemically synthesized peptides, since the N- and C-terminus are charged, acetylation of the N-terminus and / or amidation of the C-terminus are performed to remove such charges without being particularly limited thereto.

The present peptides herein may also be synthesized by methods well known in the art, for example by using automated peptide synthesizers, depending on their length, and can also be produced by genetic engineering techniques. For example, through genetic engineering, a fusion gene encoding a fusion protein comprising a fusion partner and a peptide of the present invention is prepared and transformed into a host cell, and then expressed in the form of a fusion protein, Then the peptide of the present invention may be cleaved and separated from the fusion protein to produce a desired peptide using a protease or compound.

The epitope according to the present disclosure specifically recognize anti-AQP5 autoantibodies (also called AQP5 autoantibodies).

Sjogren's syndrome is a disease with major symptoms of dry mouth and dry eye caused by disfunction of the salivary and lacrimal glands due to autoimmune reactions. In the serum of early stage of Sjogren syndrome patients, anti-AQP1 and anti-AQP5 autoantibodies are present, and the antigens of the antibodies AQP1 and AQP5, are members of waterway protein family (AQP 12) expressed in salivary glands and lacrimal glands. The binding of autoantibodies to the extracellular loop A and C regions as well as the extracellular loop E forming the water pathway of AQP5 can prevent the passage of water through the water passage. When the AQP5 autoantibody binds to AQP5, water molecules are prevented from passing through the cell membrane, thereby reducing saliva and tear secretion. Thus, the activity of anti-aquaporin 5 autoantibody may be one of the causes of Sjogren's syndrome.

Thus, in other aspect, the present disclosure relates to compositions comprising the present epitopes for detecting AQP5 autoantibodies, and methods of detecting AQP5 autoantibodies using the composition.

The term detection herein includes quantitative and/or qualitative analysis, including the detection of presence, absence, and measurement of concentration. Such methods are known in the art and those skilled in the art can select appropriate methods for the practice of the present application.

The present epitopes of the present disclosure can be particularly useful for the diagnosis of Sjogren's syndrome.

As used herein, the term "diagnosis" refers to determining the susceptibility of a subject, to a particular disease or condition, determining whether an subject currently has a particular disease or condition, and determining the prognosis of a subject with a disease or condition (e.g., to determine the stage of progression or to determine the responsiveness of the disease to treatment) or therametrics (e.g., monitoring the status of a subject to provide information about treatment efficacy).

Thus, in other aspect, the present disclosure relates to a method of detecting AQP5 to diagnosis or to provide information as to the diagnosis of Sjogren's syndrome comprising the steps of: providing at least one of the present epitopes; providing a sample provided from a subject in need of detection of AQP5 autoantibody; contacting the sample and the polypeptide by which a complex between the two is formed; and detecting the complex formation of the epitope and the autoantibody, wherein the positive detection results, for example, the presence of the complex, or the increase in the amount of the complex, in comparison to a normal healthy sample indicates that the sample or the subject is inflicted with Sjogren's syndrome.

The sample or biological sample according to the present invention is a sample provided from a subject in need of diagnosis, progression of the disease, or detection of Aquaporin 5 autoantibody of Sjogren's syndrome, for example, a person suspected of Sjogren's syndrome or a person with Sjogren's syndrome or a person being treated after diagnosis with Sjogren's syndrome, but is not limited thereto.

Such samples are not particularly limited as long as they can be used for detecting aquaporin 5 autoantibodies, and refer to a material or a mixture of materials that include one or more detectable components, such as cells, tissues or body fluids derived from living organisms, in particular humans. It includes but is not limited to saliva, body fluids such as tears, whole blood, plasma, and serum. In one embodiment according to the invention blood, in particular serum, is used.

In the method according to the invention, the antigen-antibody complex can be detected using various known methods. For example, the detection of the complex may be performed by Radial Immunodiffusion, Immunoprecipitation including Reverse Current Electrophoresis and Immunoelectrophoresis, Competition indirect immunofluorescent assay or ELISA(Enzyme Linked Immunosorbent Assay. Detection in this kind of methods is particularly advantageous when the antigen is provided as a soluble protein. In one embodiment according to the present application, an ELISA method, in particular, a sandwich type ELISA is used, in which case the detection antibody described below is also used.

In other embodiments, when the antigen is provided in the form of cells, cell-based assay methods may be used, for example, FACS (fluorescence-activated cell sorting) assay, or cellular immunostaining with antibody, for example, immunofluorescence cell staining as described in examples of the present disclosure.

Through the process of detecting the antigen-antibody complex, the intensity of the final signal of the sample is analyzed and compared with that of the signal of a normal control sample. When the signal intensity is higher than that of the normal control sample, the sample may be diagnosed as Sjogren's syndrome, or treatment progress may be determined.

In one embodiment of the present disclosure, for detection of the antigen-antibody complex, the antigen is labeled with a label as described below, or a detection antibody binding human Ig may be labelled.

The detection antibody that may be used in the method according to the present disclosure binds specifically to human IgG or IgA. The detection antibody may be labeled with a substance that allows a visual detection or a detection by use of various image detection systems.

In one embodiment of the present disclosure, the epitope or detection antibody according to the present disclosure may be labeled with an enzyme that can catalyze a chemical reaction in the presence of a specific substrate to produce a color reaction or emit light. Examples of the enzyme include, but are not limited to, peroxidases such as horseradish peroxidase, alkaline phosphatase, glucose oxidase, beta-galactosidase, urease, catalase, asparginase, ribonuclease, malate dehydrogenase, staphylococcal nuclease, triosephosphate isomerase, glucose-6-phosphate dehydrogenase, glucoamylase, and acetylcholine esterase.

In other embodiments, the antigen or detection antibody according to the present disclosure may be labeled with a chromophore that emits light having a wavelength different from that of irradiated light by light irradiation and that is used in bioluminescence, chemiluminescence, electroluminescence, electrochemiluminescence, and photoluminescence. Examples of the chromophore include, but are not limited to, proteins, including green fluorescence protein; and organic compounds, including fluorescein isothiocyanate, rhodamine, phycoerythrin, phycocyanin, allophycocyanin, and fluorecamine.

In another embodiment, the antigen or detection antibody according to the present disclosure may be labeled with various radioisotopes. In the present disclosure, when the label is, for example, a radioisotope, detection of the label may be performed by a scintillation counter, and when the label is a fluorescent substance, detection of the label may be performed by a method such as spectroscopy, a phosphoimaging system or a fluorescence counter. When the label is an enzyme, detection of the label may be performed by measuring a chromogenic product that occurs by enzymatic conversion of a suitable chromogenic substrate. Furthermore, detection may be performed by comparing the color of a chromogenic product that occurs by an enzymatic reaction, through comparison with a suitable standard or control.

In one embodiment, the antigen or detection antibody according to the present disclosure comprises, for example, a chromophore; an enzyme including alkaline phosphatase, biotin, beta-galactosidase or peroxidase; a radioisotope; or a material comprising nanoparticles such as colloidal gold particles or colored latex particles, but is not limited thereto.

In another reference aspect, there is provided a kit for detecting or diagnosing Sjogren's syndrome, which is used in the method according to the present disclosure and comprises a solid support having the present epitope adsorbed or attached thereon and a detection antibody, in which the detection antibody binds specifically to human IgG, and the antigen or the detection antibody is labeled with a chromophore; an enzyme including alkaline phosphatase, biotin, beta-galactosidase or peroxidase; a radioisotope; or a material comprising nanoparticles, including colloidal gold particles or colored latex particles.

Among the components that are included in the kit, reference may be made to the foregoing description.

In the kit, the epitopes according to the present disclosure may be attached to a microwell plate such as a 96-well microwell plate, beads or particles, including colloidal gold particles or colored latex particles, or a membrane such as a cellulose, nitrocellulose, polyethersulfone, polyvinylidene, fluoride, nylon, charged nylon or polytetrafluoroethylene membrane. For attachment or coating of the antigen, a known method may be used. For example, attachment or coating of the antigen may be performed with reference to the method descried in examples of the present disclosure.

In one embodiment, the method may be used in a sandwich-type immunoassay such as ELISA (Enzyme Linked Immuno Sorbent assay), RIA (Radio Immuno Assay) or the like. In this method, a sample may be contacted with an antigen bound to a solid support, for example, a bead, membrane, slide or microwell plate made of glass, plastic (e.g., polystyrene), polysaccharide, nylon or nitrocellulose, and then an antigen-antibody complex may be qualitatively or quantitatively detected by an antibody either labeled with a directly or indirectly detectable label, for example, a radioisotope such as ³H or ¹²⁵I as described above, a fluorescent substance, a chemiluminescent substance, heptene, biotin, digoxigenin or the like, or conjugated with an enzyme such as horseradish peroxidase, alkaline phosphatase or malate dehydrogenase, which can develop color or emit light by its reaction with a substrate. Furthermore, the immunoassay method is described in Enzyme Immunoassay, E. T. Maggio, ed., CRC Press, Boca Raton, Florida, 1980; Gaastra, W., Enzyme-linked immunosorbent assay(ELISA), in Methods in Molecular Biology, Vol. 1, Walker, J.M. ed., Humana Press, NJ, 1984, etc. The ELISA kit may further comprise reagents capable of detecting the bound antibody, for example, a secondary detection antibody labeled with the above-described substance such as a chromophore, an enzyme (e.g., conjugated with antibody) or the like, and a substrate that is used in detection.

The method or kit according may be used in the form of arrays, including microarrays, or chips. The antigen may be attached to the surface of a support such as glass or nitrocellulose. For array fabrication technology, reference may be made to, for example, Schena et al., 1996, Proc Natl Acad Sci USA. 93(20):10614-9; Schena et al., 1995, Science 270(5235):467-70; and U.S. Pat. No. 5,599,695, 5,556,752 or 5,631,734. As a fluorescence spectrometer, a scanning confocal microscope may be used and is available from, for example, Affymetrix, Inc. or Agilent Technologies, Inc.

In addition, the method or kit is used in the form of a dip stick rapid kit depending on the pattern of analysis. The dip stick rapid kit is a technology that is widely used in the field of POCT (Point of Care Treatment). In the case of the dip stick rapid kit, the antigen according to the present disclosure is bound to a support such as nitrocellulose, and when the antigen is brought into contact with a sample (for example, one end of the dip stick is dipped in a serum sample), the sample moves a substrate by a capillary phenomenon so that the antigen will bind to an antibody in the substrate to develop color. In this manner, the antigen-antibody complex is detected.

Furthermore, the kit may be used in a lateral flow assay depending on the pattern of analysis. The lateral flow assay is a method for quantitatively or qualitatively measuring a specific substance (e.g., a specific nucleic acid or protein) contained in a sample. For example, in the lateral flow assay, using a nitrocellulose membrane (medium for development) having an antigen bound at a specific position, the substance to be analyzed is moved by a chromatographic method, and a specific nucleic acid or protein in the sample is detected by an antigen-antibody reaction.

Furthermore, the kit may further comprise a guidebook about a method of using the present epitopes according to the present disclosure. In addition, the kit may further comprise a reagent or the like, which can detect an antibody specific for a control, or a bound antibody.

The present inventors also endeavored to find the epitopes that can detect AQP5 autoantibodies that can affect the function of AQP5 as well as can reverse the inhibitory effect by the autoantibodies restoring the function AQP5 of for therapeutic use.

Thus, in other aspect, the present invention relates to a pharmaceutical composition for treating Sjogren's syndrome, comprising at least one of the epitope peptides of SEQ ID NO: 1 to 6.

The epitope peptides of SEQ ID NO: 1 to 6 specifically recognize anti-AQP5 autoantibodies, thereby binding to and neutralizing anti-AQP5 autoantibodies through antigen-antibody reactions. Therefore, the present epitope can be effectively used to treat Sjogren's syndrome by preventing the inhibitory activity or reversing the suppression of the secretion by the anti-AQP5 autoantibodies.

As used herein, the term "treatment" includes inhibiting, eliminating, alleviating, ameliorating or improving a disease or condition or condition resulting from the disease.

The composition of the present invention may be prepared by including one or more pharmaceutically acceptable carriers in addition to the above-mentioned active ingredients. Pharmaceutically acceptable carriers may be used in combination with saline, sterile water, Ringer's solution, buffered saline, dextrose solution, maltodextrin solution, glycerol, ethanol, liposomes, and one or more of these components, as necessary. Other conventional additives such as buffers and bacteriostatic agents can be added. In addition, diluents, dispersants, surfactants, binders and lubricants may be additionally added to formulate into injectable formulations, pills, capsules, granules or tablets such as aqueous solutions, suspensions, emulsions, and the like, and may be prepared to act specifically on target organs, in which case, target organ specific antibodies or other ligands may be used in combination with the carriers. Furthermore, it may be preferably formulated according to specific condition or component using an appropriate method in the art or using the method disclosed in Remington's Pharmaceutical Science (Latest Edition, Mack Publishing Company, Easton PA).

The administration route of the composition of the present application is not particularly limited, and known administration methods may be applied, and parenteral administration (for example, intravenous, subcutaneous, intraperitoneal, or topical) may be used depending on the desired method. Administration by intravenous injection is preferred, in order to obtain a rapid therapeutic effect. The dosage may vary depending on the age, weight, sex, dosage form, health condition and degree of disease of the subject, and is generally 0.01 to 1,000 mg / day, based on an adult patient weighing 70 kg. Preferably it is 0.01-500 mg / day, and can be divided into once or several times a day.

In another aspect, the present invention relates to a Sjogren's syndrome diagnostic composition comprising at least one of the epitope of SEQ ID NO: 1 to 6.

The composition according to the present application can be used for determining the onset, the progression or the diagnosis of Sjogren's syndrome.

SEQ ID NO: 1, 3 or 6 peptide antigens may be provided in the form of cells expressing it, in particular such cells that do not express any aquaporin including aquaporin 5 may be used. In the case of cells not expressing aquaporin 5, a plasmid expressing aquaporin 5 may be transferred to the cells for exogenous expression of AQP5. For example, MDCK cells as described in the Examples herein can be used, but are not limited thereto. Aquaporin 5 is a transmembrane protein present in cell membranes that is difficult to maintain its conformation or folded structure when present in aqueous solutions other than lipid membranes, which may makes the detection of antibodies that bind to AQP5 expressed in salivary or lacrimal glands.

The sample or biological sample according to the present application is a sample derived from a person suspected of Sjogren's syndrome, a person suffering from Sjogren's syndrome, or a person being diagnosed with Sjogren's syndrome and being treated.

Such samples are not particularly limited as long as they can be used for detecting aquaporin 5 autoantibodies, and refer to a material or a mixture of materials that include one or more detectable components, such as cells, tissues or body fluids derived from living organisms, in particular humans. It includes but is not limited to saliva, body fluids such as tears, whole blood, plasma, and serum. In one embodiment according to the invention blood, in particular serum, is used.

In the method according to the present disclosure, the antigen-antibody complex can be detected using various methods known in the art. For example, the detection may be performed by Radial Immunodiffusion, Immunoprecipitation including Reverse Current Electrophoresis and Immunoelectrophoresis, Competition indirect immunofluorescent assay or ELISA(Enzyme Linked Immunosorbent Assay. Detection in this kind of methods is particularly advantageous when the antigen is provided as a soluble protein. In one embodiment according to the present application, an ELISA method, in particular, a sandwich type ELISA is used, in which case the detection antibody described below is also used.

In other embodiments, when the antigen is provided in the form of cells, cell-based assay methods may be used, for example, FACS (fluorescence-activated cell sorting) assay, or cellular immunostaining with antibody, for example, immunofluorescence cell staining as described in examples of the present disclosure.

Through the process of detecting the antigen-antibody complex, the intensity of the final signal of the sample is analyzed and compared with that of the signal of a normal control sample. When the signal intensity is higher than that of the normal control sample, the sample may be diagnosed as Sjogren's syndrome, or progress in the treatment may be determined.

In one embodiment of the present disclosure, for detection of the antigen-antibody complex, the antigen is labeled with a label as described below, or a detection antibody or secondary antibody is employed.

The detection antibody that may be employed in the method according to the present disclosure binds specifically to human IgG or IgA. The detection antibody may be labeled with a substance that can be detected visually or using various image detection systems.

In one embodiment of the present disclosure, the antigen or detection antibody according to the present disclosure may be labeled with an enzyme that can catalyze a chemical reaction in the presence of a specific substrate to produce a color reaction or emit light. Examples of the enzyme include, but are not limited to, peroxidases such as horseradish peroxidase, alkaline phosphatase, glucose oxidase, beta-galactosidase, urease, catalase, asparginase, ribonuclease, malate dehydrogenase, staphylococcal nuclease, triosephosphate isomerase, glucose-6-phosphate dehydrogenase, glucoamylase, and acetylcholine esterase.

In other embodiments, the antigen or detection antibody according to the present disclosure may be labeled with a chromophore that emits light having a wavelength different from that of irradiated light by light irradiation and that is used in bioluminescence, chemiluminescence, electroluminescence, electrochemiluminescence, and photoluminescence. Examples of the chromophore include, but are not limited to, proteins, including green fluorescence protein; and organic compounds, including fluorescein isothiocyanate, rhodamine, phycoerythrin, phycocyanin, allophycocyanin, and fluorecamine.

In another embodiment, the antigen or detection antibody according to the present disclosure may be labeled with various radioisotopes.

In the present disclosure, when the label is, for example, a radioisotope, detection of the label may be performed by a scintillation counter, and when the label is a fluorescent substance, detection of the label may be performed by a method such as spectroscopy, a phosphoimaging system or a fluorescence counter. When the label is an enzyme, detection of the label may be performed by measuring a chromogenic product that occurs by enzymatic conversion of a suitable chromogenic substrate. Furthermore, detection may be performed by comparing the color of a chromogenic product that occurs by an enzymatic reaction, through comparison with a suitable standard or control.

In one embodiment, the antigen or detection antibody according to the present disclosure comprises, for example, a chromophore; an enzyme including alkaline phosphatase, biotin, beta-galactosidase or peroxidase; a radioisotope; or a material comprising nanoparticles such as colloidal gold particles or colored latex particles, but is not limited thereto.

In another reference aspect, there is provided a kit comprising at least one of the polypeptides of SEQ ID NO: 1 to 6 for detecting or diagnosing Sjogren's syndrome. The kit is used in the method according to the present disclosure and further comprises a solid support having the present epitopes adsorbed thereon and a detection antibody, in which the detection antibody binds specifically to human IgG, and the antigen or detection antibody is labeled with a chromophore; an enzyme including alkaline phosphatase, biotin, beta-galactosidase or peroxidase; a radioisotope; or a material comprising nanoparticles, including colloidal gold particles or colored latex particles.

Among the components that are included in the kit and that overlap with the above-described components, reference may be made to the foregoing description.

In the kit according to the present disclosure, the epitopes according to the present disclosure may be attached to microwell plates such as a 96-well microwell plate, beads or particles including colloidal gold particles or colored latex particles, or membranes such as a cellulose, nitrocellulose, polyethersulfone, polyvinylidene, fluoride, nylon, charged nylon or polytetrafluoroethylene membrane. The attachment of or coating with the epitopes can be achieved by methods known in the art. For example, the attachment of or coating with the epitopes may be performed with reference to the method descried in examples of the present disclosure.

In one embodiment, the method according to the present disclosure may be used in a sandwich-type immunoassay such as ELISA (Enzyme Linked Immuno Sorbent assay), RIA (Radio Immuno Assay) or the like. In this method, a sample may be added to an antigen bound to a solid support, for example, a bead, membrane, slide or microwell plate made of glass, plastic (e.g., polystyrene), polysaccharide, nylon or nitrocellulose, and then an antigen-antibody complex may be qualitatively or quantitatively detected by antigen binding to an antibody either labeled with a directly or indirectly detectable label, for example, a radioisotope such as ³H or ¹²⁵I as described above, a fluorescent substance, a chemiluminescent substance, heptene, biotin, digoxigenin or the like, or conjugated with an enzyme such as horseradish peroxidase, alkaline phosphatase or malate dehydrogenase, which can develop color or emit light by its reaction with a substrate. Furthermore, the immunoassay method is described in Enzyme Immunoassay, E. T. Maggio, ed., CRC Press, Boca Raton, Florida, 1980; Gaastra, W., Enzyme-linked immunosorbent assay (ELISA), in Methods in Molecular Biology, Vol. 1, Walker, J.M. ed., Humana Press, NJ, 1984, etc. The ELISA kit may further comprise reagents capable of detecting the bound antibody, for example, a secondary detection antibody labeled with the above-described substance such as a chromophore, an enzyme (e.g., conjugated with antibody) or the like, and a substrate that is used in detection.

In other embodiments, the method according to the present disclosure may be used in the form of arrays, including microarrays, or chips. The antigen may be attached to the surface of a support such as glass or nitrocellulose. For array fabrication technology, reference may be made to, for example, Schena et al., 1996, Proc Natl Acad Sci USA. 93(20):10614-9; Schena et al., 1995, Science 270(5235):467-70; and U.S. Pat. No. 5,599,695, 5,556,752 or 5,631,734. As a fluorescence spectrometer, a scanning confocal microscope may be used and is available from, for example, Affymetrix, Inc. or Agilent Technologies, Inc.

In addition, the method or kit according to the present disclosure is used in the form of a dip stick rapid kit depending on the pattern of analysis. The dip stick rapid kit is a technology that is widely used in the field of POCT (Point of Care Treatment). In the case of the dip stick rapid kit, the antigen according to the present disclosure is bound to a support such as nitrocellulose, and when the antigen is brought into contact with a sample (for example, one end of the dip stick is dipped in a serum sample), the sample moves a substrate by a capillary phenomenon so that the antigen will bind to an antibody in the substrate to develop color. In this manner, the antigen-antibody complex is detected.

Furthermore, the kit according to the present disclosure may be used in a lateral flow assay depending on the pattern of analysis. The lateral flow assay is a method for quantitatively or qualitatively measuring a specific substance (e.g., a specific nucleic acid or protein) contained in a sample. For example, in the lateral flow assay, using a nitrocellulose membrane (medium for development) having an antigen bound at a specific position, the substance to be analyzed is moved by a chromatographic method, and a specific nucleic acid or protein in the sample is detected by an antigen-antibody reaction.

Furthermore, the kit according to the reference aspect may comprise a guidebook about a method of using the epitopes according to the present disclosure. In addition, the kit may further comprise a reagent or the like, which can detect an antibody specific for a control, or a bound antibody.

In other aspect, the present disclosure relates to a method of detecting AQP5 autoantibodies in vitro in a sample in need thereof comprising the steps of: providing at least one of the present epitopes; providing a biological sample to be tested; contacting the epitopes with the biological sample to detect the binding between the epitopes and AQP5 autoantibodies in the sample so as to determine the presence of the autoantibodies in the sample.

In the present methods, the positive detection result, i.e., the positive for the binding between the epitopes and AQP5 autoantibodies, compared to a control indicates the sample is inflicted with Sjogren's syndrome.

In this perspective, a reference aspect relates to a method of diagnosing Sjogren's syndrome (SS) or monitoring the therapeutic efficacy after treatment or providing information for diagnosis in a subject in need thereof, the method comprising: providing at least one of the present epitopes represented by SEQ ID NO: 1 to 6; contacting a biological sample with the epitopes to allow the formation of a complex of the epitope and the autoantibodies by binding therebetween; determining whether the AQP5 autoantibody is present in the sample by detecting the complex; and diagnosing the subject having SS when the detection result is positive for the complex formation.

Among the components that are included in the methods and that overlap with the above-described components, such as the detection of complex of antigen-antibody and the like, reference may be made to the foregoing description.

The present epitopes are able to detect the AQP5 autoantibodies that can affect the function of AQP5 in cells, and to reverse the inhibitory effect by the autoantibodies which is useful for therapeutic use. There are also present in the body AQP5 autoantibodies that do not affect the function of AQP5, and the epitopes of the present disclosure can differentially detect only the autoantibodies that affect function, thus can be effectively used as therapeutic agents.

Thus, in other aspect, the present invention relates to a pharmaceutical composition for treating Sjogren's syndrome, comprising at least one of the present epitopes.

According to Fig. 3 of the present disclosure, the function of AQP5 is recovered even in the presence of AQP5 autoantibodies by treatment with the present epitopes. This clearly indicates that the present epitopes can be advantageously and effectively used for treating Sjogren's syndrome.

As described above, autoantibodies of AQP5 have been shown to be associated with the pathogenesis of Sjogren's syndrome (Robinson, C. P. et al. 1998. Transfer of human serum IgG to nonobese diabetic Igmu null mice reveals a role for autoantibodies in the loss of secretory function of exocrine tissues in Sjogren's syndrome. Proc Natl Acad Sci USA. 95: 7538-7543; Alam, J. et al.,. 2016. Detection of autoantibodies against aquaporin-5 in the sera of patients with primary Sjogren's syndrome. Immunol Res. 64:848-856.). Autoantibodies to AQP5 were detected in the serum of Sjogren's syndrome patients, and it was found that the salivation rate is significantly lower in patients with anti-AQP5 autoantibodies than that of those without AQP5 autoantibodies. Thus, this indicates that AQP5 autoantibodies are involved in the pathogenesis of Sjogren's syndrome. According to Fig. 3 of the present disclosure, the function of AQP5 is recovered even in the presence of AQP5 autoantibodies by treatment with the present epitopes. This clearly indicates that the present epitopes can be advantageously and effectively used for treating Sjogren's syndrome.

Therefore, the epitopes of the present disclosure specifically recognize and bind to AQP5 autoantibodies that affect the function of AQP5 in cells through antigen-antibody responses. The present epitopes can prevent the binding between AQP5 in cells and autoantibodies, which is useful for the treatment of Sjogren's syndrome.

As used herein, the term "treatment" includes inhibiting, eliminating, alleviating, ameliorating or improving a disease or condition or condition resulting from the disease.

The composition of the present invention may be prepared by including one or more pharmaceutically acceptable carriers in addition to the above-mentioned active ingredients. Pharmaceutically acceptable carriers may be used in combination with saline, sterile water, Ringer's solution, buffered saline, dextrose solution, maltodextrin solution, glycerol, ethanol, liposomes, and one or more of these components, as necessary. Other conventional additives such as buffers and bacteriostatic agents can be added. In addition, diluents, dispersants, surfactants, binders and lubricants may be additionally added to formulate into injectable formulations, pills, capsules, granules or tablets such as aqueous solutions, suspensions, emulsions, and the like, and may be prepared to act specifically on target organs, in which case, target organ specific antibodies or other ligands may be used in combination with the carriers. Furthermore, it may be preferably formulated according to specific condition or component using an appropriate method in the art or using the method disclosed in Remington's Pharmaceutical Science (Latest Edition, Mack Publishing Company, Easton PA).

The administration route of the composition of the present application is not particularly limited, and known administration methods may be applied, and parenteral administration (for example, intravenous, subcutaneous, intraperitoneal, or topical) may be used depending on the desired method. Administration by intravenous injection is preferred, in order to obtain a rapid therapeutic effect. The dosage may vary depending on the age, weight, sex, dosage form, health condition and degree of disease of the subject, and is generally 0.01 to 1,000 mg / day, based on an adult patient weighing 70 kg. Preferably it is 0.01-500 mg / day, and can be divided into once or several times a day.

The present disclosure is further explained in more detail with reference to the following examples. These examples, however, should not be interpreted as limiting the scope of the present invention in any manner.

### EXAMPLES

### Materials and methods

### Serum and IgG samples

This study was done in compliance with the Helsinki Declaration after approvals from the Institutional Review Board of Seoul National University Hospital (IRB number: 0912-011-302) and the Institutional Review Board of Seoul St. Mary's Hospital (IRB number: KC13ONMI0646). From our previous study (12), 13 control and 24 SS serum samples positive for anti-AQP5 IgG were chosen based on the levels of anti-AQP5 IgG and availability. The first 12 control and 12 SS samples had similar low levels of anti-AQP5 IgG, while the rest of the samples had intermediate to high levels. IgG antibodies were purified using a protein A column (ThermoFisher Scientific, Waltham, MA, USA) from the sera of 3 SS patients who were not included in the previous study. A commercial human IgG product purified from pooled normal sera (Sigma-Aldrich Korea, Seoul, Korea) was used as control. All SS patients fulfilled the 2002 American-European Consensus group classification criteria and/or the 2012 American College of Rheumatology criteria.

### EXAMPLE 1. Design of peptides

The amino acid sequence and structure of human AQP5 were subjected to prediction analysis of B cell epitopes using tools provided by the Immune Epitope Database and Analysis Resource (http://tools.immuneepitope.org/main/bcell/). Six peptide sequences chosen from the three extra cellular loops and the second α-helix of AQP5 were synthesized in linear or cyclic forms (Peptron Inc., Daejeon, Korea). A peptide of irrelevant sequence (Sigma-Aldrich) was also used as control (Table 1).

**[Table 1] The amino acid sequence of the epitope of the present disclosure and their location in AQP5 protein.**

| Name | Position in AQP5 | AA Sequence | Type | SEQ ID NO |
|---|---|---|---|---|
| A | Extracellular loop A aa 35-41 | WPSALPT | Linear | 1 |
| A2 | Extracellular loop A aa 35-41 | **C**WPSALPT**C** | Cyclic | 2 |
| H(C) | Transmembrane helix 2 aa 48-55 | AFGLAIGT | Linear | NA |
| C1 | Extracellular loop C aa 123-129 | **C**NNNTTQG**C** | Cyclic | 3 |
| C2 | Extracellular loop C aa 123-129 | NNNTTQG | Linear | 4 |
| E1 | Extracellular loop E aa 181-191 | G**C**SMNPARSFG**C** | Cyclic | 5 |
| E2 | Extracellular loop E aa 184-191 | **C**MNPARSFG**C** | Cyclic | 6 |
| E3 | aa 185-191 | NPARSFG | Linear | 7 |
| IRP(C) | Irrelevant Sequence | GRADSP | Linear | NA |

In Table 1, a double bond is formed between the two cysteines marked in bold resulting in forming a circular structure, in which the cysteines underlined were artificially introduced in the present disclosure to form a double bond. In Table 1, (C) indicate a control, NA (Not applicable) indicates that it is not listed in the sequence listing.

### EXAMPLE 2. Indirect immunofluorescence (IIF) assay

Madin-Darby canine kidney (MDCK) cells expressing full-length human AQP5 (MDCK-AQP5) were maintained in DMEM medium with 10% FBS and 1% penicillin/streptomycin in the presence of G418 (12). A mixture of non-transfected MDCK and MDCK-AQP5 cells (6 × 10⁴) was plated onto collagen-coated coverslips of 12 mm diameter. After stimulation with 0.5 mM cAMP for 24 hours, the cells were fixed with 4% paraformaldehyde in PBS (pH 7.4) and subjected to antigen retrieval by incubation in sodium citrate buffer (10 mM sodium citrate, 0.05% Tween-20, pH 6) at 105 °C for 20 min. After blocking with 5% BSA in PBS, the cells were incubated with goat polyclonal antibodies raised against a peptide near the C-terminus of human AQP5 (Santa Cruz, Paso Robles, CA, USA) along with control/SS sera (1:200 dilution) or purified IgG (10 µg) that were pre-incubated overnight with 10 µg synthetic peptides in RIA buffer (10 mM Tris-HCl, 350 mM NaCl, 1% BSA, 1% Triton X-100, 0.5 % Sodium deoxycholate, 0.1% SDS, 10% Horse serum, pH 7.6). Subsequently, the cells were stained with Alexa Fluor 488-conjugated donkey anti-goat IgG (Invitrogen, Carlsbad, CA, USA) and CF^{™} 594-conjugated rabbit anti-human IgG (Sigma-Aldrich). At least four images of AQP5-expressing cells were randomly selected, based on the goat anti-AQP5 antibody stain, and imaged sequentially for the staining with human IgG using a confocal microscope (Carl Zeiss, Jena, Germany). After coding the images, two individuals blindly determined the relative intensities of the anti-AQP5 human IgG signals by decreasing the brightness of the red signal until all the signals of AQP5 stain disappeared. When the values of the four images did not pass an one-sample t-test with hypothetical mean intensity of 0, additional images were obtained. The mean of the Δ brightness was used to express the anti-AQP5 IgG level in each sample.

The differences in proportion between two groups were determined by Fisher's exact test. Differences in means between the experimental and control groups were determined by Student's t-test, while the differences among peptides were determined by Oneway ANOVA. All statistical analyses were performed using SPSS (SPSS Inc., Chicago, USA).

### EXAMPLE 3. Transepithelial water flow measurement

Osmotic water flow across a cell monolayer was measured by a previously described method with some modification (Levin MH et al., J Biol Chem 2006;281:25803-12). Either MDCK or MDCK-AQP5 cells (8×10⁴ cells/well) were grown on a 0.33 cm² transwell (SPL Life Science, Gyeonggi-do, Korea) for 4 days, until the transepithelial electrical resistance reaches the maximum level. The cells were incubated with purified IgG (0.2 mg/ml in PBS, 50 µl/well), non-absorbed or pre-absorbed with 20 µg of each peptide overnight, on ice for 1 hour. The monolayer was washed twice with PBS, and all the fluid was carefully removed using vacuum aspiration. After placing the transwell in 1 ml PBS, a hyperosmotic solution (300 mM D-mannitol in PBS, 200 µl/well) was added to the apical side. After incubation at 37C° for 10 or 20 minutes, the bottom of the transwell was immediately dried on paper towel, and the apical fluid was removed and measured with a micropipette. Transepithelial osmotic permeability coefficient P_{f}^{T} expressed as micrometers per second, was calculated as P_{f}^{T}=Jᵥ^{T}/Sv_{w}Δ[Φ)]^{T}, where Jᵥ^{T} is the measured transepithelial water flow (cm³/sec), S is the monolayer surface area (0.33cm²), v_{w} is the partial molar volume of water (18 cm³/mol) and Δ[□]^{T} is the transepithelial osmotic gradient (3 × 10⁻⁴ mol/cm³).

Differences in means between the experimental and control groups were determined by Student's t-test, while the differences among peptides were determined by Oneway ANOVA. All statistical analyses were performed using SPSS (SPSS Inc., Chicago, USA).

### EXAMPLE 4. Design of peptides mimicking the epitopes of AQP5

Stretches of amino acids were chosen from the region surrounding the three extracellular loops by referring to the results of B cell epitope prediction tools that employ algorithms based on the sequence of protein (Fig. 1). All five loops and the C-terminal cytoplasmic region presented scores above the threshold for five parameters; however, the prediction result on the Kolaskar & Tongaonkar antigenicity scale was quite different from the others. In particular, NNNTTQG (peptide C) from loop C presented the highest score for Chou & Fasman beta-turn and Karplus & Schulz flexibility throughout the entire sequence, and was also positive for other parameters except for Kolaskar & Tongaonkar antigenicity. WPSALPT (peptide A), chosen from loop A, was positive for all six parameters. From loop E, long (peptide E1) and short (peptides E2/E3) sequences containing the NPA motif were chosen, which presented higher scores than peptide A in most parameters except for the Kolaskar & Tongaonkar antigenicity. According to the result of ElliPro analysis, which employs algorithms based on the structure of protein, only the sequences chosen from loops A and C were included in the list of linear epitopes. As a control, a sequence from the second transmembrane helix (peptide H) was chosen, which was negative for all seven parameters (Fig. 1). Aside from peptide A which failed to synthesize in a cyclic form, both linear and cyclic forms were tested for peptides C and E. A linear peptide of irrelevant sequence (IRP) was also included as a control (Table 1).

### EXAMPLE 5. Difference in the fine specificity of anti-AQP5 autoantibodies between control subjects and SS patients

To analyze the epitopes recognized by anti-AQP5 autoantibodies, MDCK-AQP5 cells were double stained with goat anti-AQP5 antibodies and serum from either control subjects or SS patients that were pre-incubated with each peptide (Fig. 2A). The intensity of AQP5 staining by human IgG in the presence of each peptide was expressed as the percentage of control conditions in the absence of peptides (Table 2). Because the normalized intensities of AQP5 staining in the presence of IRP varied from 69 to 173 depending on the sample, signal intensities smaller than 62.2, which is the mean - 2SD of values in the presence of IRP, were regarded as being inhibited by the corresponding peptide.

Among the 24 SS serum samples, 23 were inhibited by at least one peptide, and 16 of the 23 samples recognized more than one site among the second helix and the loops A, C, and E. In contrast, only 7 out of 13 control serum samples were inhibited by any peptide, with 2 of the 7 samples recognizing multiple sites, which resulted in significant differences from the SS group (*p* = 0.004 and *p* = 0.005, respectively). Similarly, AQP5-staining by purified SS-IgG was inhibited by multiple peptides, while control-IgG was not inhibited by any peptide (Table 2 and Fig. 2A). Notably, the peptides inhibited only the staining of AQP5 with SS autoantibodies, but not that of nuclear antigens (Fig. 2A).

Among the seven peptides originating from the AQP5 sequence, E1 was the most frequently recognized one, inhibiting 20 of 27 SS and 2 of 14 control samples. It also best differentiated between SS and control samples. Peptides A, C2, and E2 were also more frequently recognized by SS than by control samples (Table 2). By comparison of the mean signal intensities, peptides A, C1, C2, E1, and E2 inhibited anti-AQP5 autoantibodies from the SS group by 40 to 50%, whereas anti-AQP5 autoantibodies from the control group were significantly inhibited only by peptides E1 and E2 (Fig. 2B). Interestingly, peptides E3 and H often increased the staining of AQP5, especially by the control group's sera (Fig. 2A and B).

When the efficiencies of linear vs. cyclic forms of peptides were compared, the epitope chosen from loop C did not show a significant difference (p = 0.163). In contrast, the epitopes from loop E worked well only in cyclic forms (Table 2 and Fig. 2B).

Collectively, these results indicate that the anti-AQP5 autoantibodies from the SS group recognize multiple extracellular epitopes more frequently than those from the control group.

In the previous study by the present inventor in which whole AQP5 proteins were used as an antigen, anti-AQP5 autoantibodies were detected in 72% of SS sample and 32% of the control sample. Interestingly, only half of the control samples recognized extracellular epitopes located on loops A, C, and E, compared to 89% for the SS samples.

There are two potential reasons for the observed difference in the frequency of extracellular epitopes found between control and SS samples. The first possibility is that the inhibitory effects of peptides on anti-AQP5 autoantibodies failed to be detected in the IIF assay due to the low levels of autoantibodies in control samples. However, this is not likely the case because 11 out of the 12 SS serum samples with similar low levels of anti-AQP5 autoantibodies presented reduced AQP5 staining in the presence of peptides. Alternatively, the anti-AQP5 autoantibodies present in control samples may recognize intracellular rather than extracellular epitopes. None of the control samples recognized the epitope chosen from the second transmembrane alpha helix, which was recognized by only two SS samples. This low frequency of peptide H recognition coincides with the results of B cell epitope prediction. There is a high possibility of the presence of the anti-AQP5 autoantibodies that preferentially recognize cytoplasmic epitopes, which showed high scores in B cell epitope prediction, was not tested.

Another difference in the fine specificities of anti-AQP5 antibodies between control and SS was the fact that SS samples, but not control, often recognized multiple epitopes. The production of antibodies binding to loops A and C via epitope spreading may have occurred preferentially in SS patients

In contrast to loops A and C, the linear E3 peptide was not able to mimic loop E although its sequence is 100% conserved in the AQPZ/porin of two oral bacterial species. According to the structure of AQP5, the NPA motif is oriented towards the cytoplasm while the R188 side chain is oriented towards the extracellular medium. Cyclization of peptides E1 and E2 by a cysteine double bond may place G191 close to R188, mimicking the epitope on the native protein (Fig. 4)

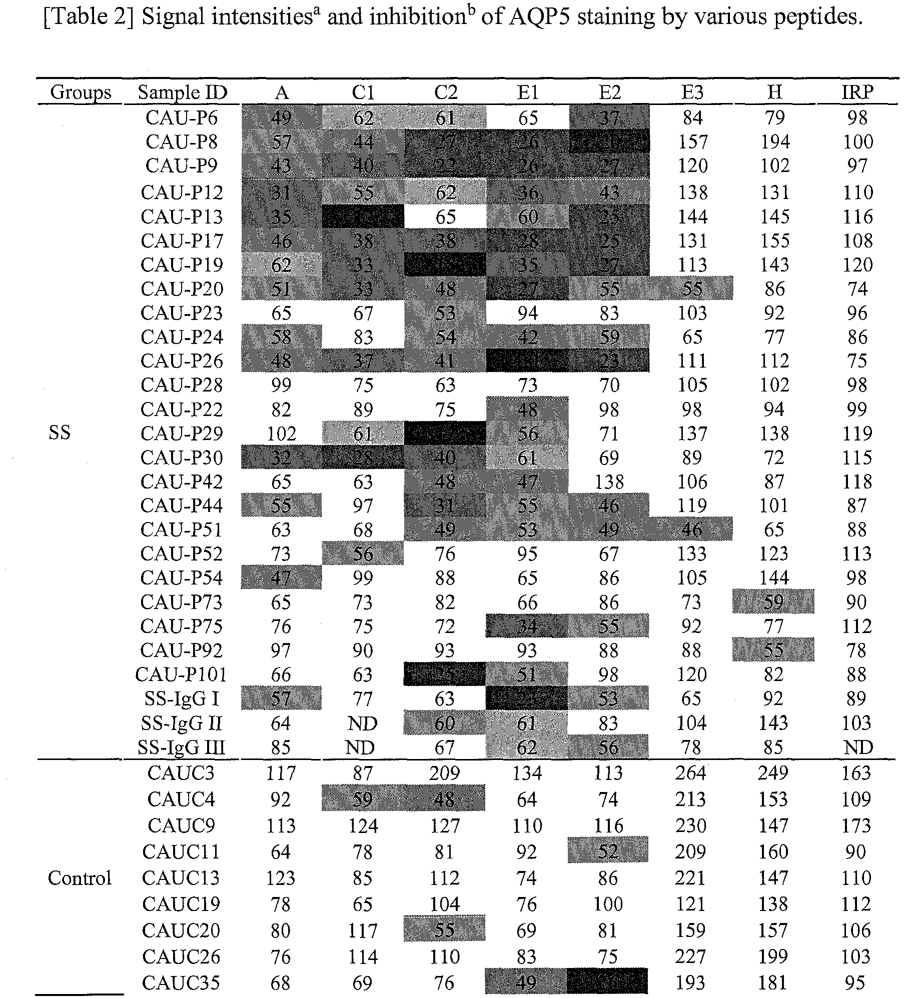

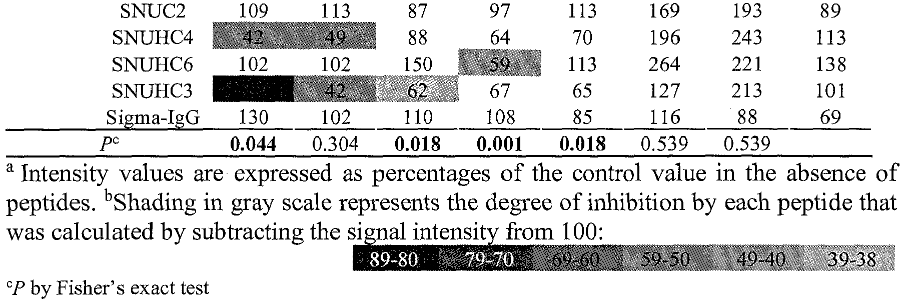

### EXAMPLE 6. Interference of function by anti-AQP5 autoantibodies that bind to the extracellular loops of AQP5

To determine the functional epitopes of AQP5, the effects of anti-AQP5 autoantibodies on osmotic water permeability in MDCK-AQP5 cells were investigated in the absence or presence of peptides. Osmotically driven changes in the volume of apical solution provide quantitative measurements of water flux across the cell monolayer (Fig. 3A). Compared to non-transfected cells, MDCK-AQP5 cells increased water permeability almost four fold upon osmotic challenge (Fig. 3B). Incubation of the MDCK-AQP5 cells with purified control-IgG decreased water permeability by about 18%, but statistical significance was not achieved (Fig. 3B). In contrast, purified SS-IgG samples had significant effects on the water permeability of MDCK-AQP5 cells, decreasing it by about 32 to 53%. Pre-incubation of SS-IgG with peptides A, C2, E1, or mixture of A/C2/E1 restored the water permeability to control levels in most cases (Fig. 3D). Significant differences in neutralizing effects among different peptides were not observed.

These results indicate that binding of antibodies to any of the three extracellular loops of AQP5 interferes with the function of the water channel.

The transepithelial osmotic permeability coefficient P_{f}^{T} of MDCK and MDCK-AQP5 cells determined in the current study agrees well with those reported in literature, confirming the fidelity of the assay. Notably, the results of transepithelial water flow measurements indicated that antibodies bound not only to the pore-forming loop E but also to other extracellular loops, such as A and C, inhibit water transport through AQP5. Interestingly, blocking of autoantibody binding to any one epitope often restored the P_{f}^{T} completely, suggesting that substantial amounts of antibodies binding to functional epitopes may be needed to affect the function of AQP5. Although only four purified IgG samples were tested in the functional assay, results from the transepithelial water flow measurements corroborate well with the results from the IIF assay. Control-IgG that were not inhibited by any peptide in the IIF assay did not significantly inhibit AQP5 function.

In conclusion, the anti-AQP5 autoantibodies detected in control and SS groups showed differences in fine specificity to the functional epitopes of AQP5. Ultimately, the amounts of antibodies binding to the functional epitopes of AQP5 would decide the effects of these autoantibodies on AQP5 function. The prevalent recognition of functional epitopes by anti-AQP5 autoantibodies from SS patients suggests that anti-AQP5 autoantibodies act as mediators of glandular hypofunction and may serve as potential therapeutic targets in SS.

### EXAMPLE 7. Detection of autoantibody by ELISA using the epitopes according to the present disclosure/

In this example, anti-aquaporin 5 autoantibodies were detected by ELISA.

Existing immunofluorescence is time consuming and uses cells, requiring high technical accuracy to achieve consistent results. On the other hand, if the epitope peptides according to the present application can detect anti-aquaporin 5 autoantibodies in an ELISA, this can replace the conventional method and can be used more conveniently for diagnosis of Sjogren's syndrome.

Fig. 5 shows a schematic diagram of ELISA used in the present Example. For the experiment, Avidin (Thermo Fisher Scientific) was added to ELISA PLATE at 1 µg / 100 µl / well and coated overnight at 4 ° C. Then the wells of the plates were washed three times with PBS containing 0.01% Tween20. Subsequently, 270 µl of the Superblock Blocking Buffer (Thermo Fisher Scientific) was added to each well and removed immediately for three times. Subsequently, the epitope peptides according to the present invention labeled with biotin were diluted in 1% BSA, 100 µl of which was added to each well at 0.2 µg / well, and incubated at 25 ° C. for 1 hour. Then the wells of the plates were washed with PBS containing 0.01% Tween20 for 3 times. Subsequently, the serum of Sjogren's syndrome patient and healthy human serum were diluted 1: 200 and 1: 400 in 1% BSA, respectively and 100 µl of each were added into the wells either coated with the present epitope antigen or with avidin only as a control group and incubated for 1 hour at 25°C. Then the wells of the plates were washed three times with PBS containing 0.01% Tween20. Then Goat anti-human IgG-HRP (Abeam) was diluted 1: 2000 in 1% BSA and added to the wells and incubated at 25 ° C. for 1 hour. Then, the wells of the plates were washed with shaking 3 times for 5 minutes each in PBS containing 0.01% Tween20. Phosphate-Citrate Buffer with Sodium tablet was dissolved in tertiary distilled water, and 3,3',5,5'-Tetramethylbenzidine dihydrochloride tablet was dissolved in 10 ml of the buffer, and 100 µl of which were then added to each well and incubated at room temperature for 30 minutes. Subsequently, 50 µl of 2N H₂SO₄ was added to each well and O.D was measured at 450 nm. The amount of autoantibodies to anti-AQP5 epitopes was expressed by subtracting the OD of the only avidin-coated wells from the OD of the epitope antigen-bound wells. The results are described in FIG. 6. As shown therein, the epitope according to the present disclosure can be successfully applied to an ELISA to effectively detect autoantibodies present in a sample.

## Claims

1. A polypeptide having an amino acid sequence selected from the group consisting of GCSMNPARSFGC (SEQ ID NO: 5), CMNPARSFGC (SEQ ID NO: 6), CWPSALPTC (SEQ ID NO: 2); and CNNNTTQGC (SEQ ID NO: 3).

2. The polypeptide of claim 1, wherein the polypeptide of SEQ ID NO: 2, SEQ ID NO: 3, SEQ ID NO: 5, and SEQ ID NO: 6 has a circular structure by the bond formed between the two cysteine residues contained therein.

3. A nucleotide encoding the polypeptide of claim 1.

4. A vector comprising the nucleotide of claim 3.

5. A cell comprising the vector of claim 4.

6. An in vitro use of a composition comprising the polypeptide according to claims 1 or 2 for detecting AQP5 autoantibody.

7. A pharmaceutical composition comprising the polypeptide according to claims 1 or 2 for use in treating Sjogren's syndrome.

8. A method for detecting AQP5 autoantibody comprising the steps of:
Providing the polypeptide according to claims 1 or 2;
Providing a sample that has been obtained from a subject in need of detection of AQP5 autoantibody; and
Contacting the sample with the polypeptide to detect a binding of AQP5 autoantibody and the polypeptide and to correlate the detection results with the presence of AQP5 autoantibody in the sample.

9. The method of claim 8, wherein the positive detection result compared to a control indicates the sample is inflicted with Sjogren's syndrome.

10. The method of claims 8 or 9, wherein the sample is a whole blood, a serum, a plasma, a tear or a saliva.

11. The method of any one of claims 8 to 10, wherein the detection is performed by FACS (Fluorescence-activated cell sorting), Cell immunostaining, Radial Immunodiffusion, Immunoelectrophoresis, Immunoprecipitation includig Reverse Current Electrophoresis, RIA (Radioimmunoassay) or ELISA (Enzyme Linked Immunosorbent Assay).

12. The method of any one of claims 8 to 10,
wherein the binding between the polypeptide and the autoantibody forms a complex and the detection includes a use of a detection antibody which specifically recognizes the autoantibody in the complex, and
wherein the detection antibody specifically recognizes human IgG or IgA and is labelled with chromophores including an enzyme including alkaline phosphatase, biotin, beta-galactosidase or peroxidase; radioactive material; or a material including color particles including colloidal gold particles or color latex particles.

13. The method of any one of claims 8 to 10,
wherein the polypeptide is labelled with a biotin,
wherein the step of detecting the binding of the polypeptide and the autoantibody further comprises the steps of contacting the biotin labelled polypeptide with a solid substrate labelled with avidin or streptavidin; applying the sample to the solid substrate to allow a binding between the autoantibody of the sample and the polypeptide attached to the solid substrate; and apply a detection antibody that specifically detect the autoantibody bound to the polypeptide, wherein the detection antibody specifically recognizes a human IgG or IgA and is labelled with chromophores including an enzyme including alkaline phosphatase, biotin, beta-galactosidase or peroxidase; radioactive material; or a material including color particles including colloidal gold particles or color latex particles.

14. An in vitro use of a peptide for detecting AQP5 autoantibody, wherein the peptide has an amino acid sequence selected from the group consisting of GCSMNPARSFGC (SEQ ID NO: 5), CMNPARSFGC (SEQ ID NO: 6), CWPSALPTC (SEQ ID NO: 2); and CNNNTTQGC (SEQ ID NO: 3).

## Patentansprüche

1. Polypeptid mit einer Aminosäuresequenz ausgewählt aus der Gruppe bestehend aus GCSMNPARSFGC (SEQ ID NO: 5), CMNPARSFGC (SEQ ID NO: 6), CWPSALPTC (SEQ ID NO: 2); und CNNNTTQGC (SEQ ID NO: 3).

2. Polypeptid nach Anspruch 1, wobei das Polypeptid von SEQ ID NO: 2, SEQ ID NO: 3, SEQ ID NO: 5 und SEQ ID NO: 6 durch die zwischen den zwei darin enthaltenen Cvsteinresten gebildete Bindung eine kreisförmige Struktur aufweist.

3. Nucleotid, das für das Polypeptid nach Anspruch 1 kodiert.

4. Vektor, umfassend das Nukleotid nach Anspruch 3.

5. Zelle, umfassend den Vektor nach Anspruch 4.

6. In-vitro-Verwendung einer das Polypeptid nach Anspruch 1 oder 2 umfassenden Zusammensetzung zur Detektion eines AQP5-Autoantikörpers.

7. Pharmazeutische Zusammensetzung, umfassend das Polypeptid nach Anspruch 1 oder 2 zur Verwendung bei der Behandlung des Sjogren-Syndroms.

8. Verfahren zur Detektion eines AQP5-Autoantikörpers, das die folgenden Schritte umfasst:
Bereitstellen des Polypeptids nach Anspruch 1 oder 2;
Bereitstellen einer Probe, die von einem Subjekt erhalten wurde, das die Detektion eines AQP5-Autoantikörpers benötigt; und
Kontaktieren der Probe mit dem Polypeptid, um eine Bindung des AQP5-Autoantikörpers und des Polypeptids zu detektieren und die Detektionsergebnisse mit dem Vorhandensein eines AQP5-Autoantikörpers in der Probe zu korrelieren.

9. Verfahren nach Anspruch 8, wobei das positive Detektionsergebnis im Vergleich zu einer Kontrolle anzeigt, dass die Probe mit dem Sjogren-Syndrom behaftet ist.

10. Verfahren nach Anspruch 8 oder 9, wobei die Probe ein Vollblut, ein Serum, ein Plasma, eine Träne oder ein Speichel ist.

11. Verfahren nach einem der Ansprüche 8 bis 10, wobei die Detektion durch FACS (Fluoreszenz-aktivierte Zellsortierung), Zell-Immunfärbung, Radiale Immundiffusion, Immunelektrophorese, Immunpräzipitation einschließlich Umkehrelektrophorese, RIA (Radioimmunoassay) oder ELISA (Enzyme Linked Immunosorbent Assay) durchgeführt wird.

12. Verfahren nach einem der Ansprüche 8 bis 10,
wobei die Bindung zwischen dem Polypeptid und dem Autoantikörper einen Komplex bildet und die Detektion eine Verwendung eines Detektionsantikörpers einschließt, der den Autoantikörper in dem Komplex spezifisch erkennt, und
wobei der Detektionsantikörper spezifisch menschliches IgG oder IgA erkennt und mit Chromophoren markiert ist, die ein Enzym, das alkalische Phosphatase, Biotin, beta-Galaktosidase oder Peroxidase einschließt, radioaktives Material oder ein Material, das Farbpartikel einschließlich kolloidaler Goldpartikel oder Farblatexpartikel einschließt, einschließen.

13. Verfahren nach einem der Ansprüche 8 bis 10,
wobei das Polypeptid mit einem Biotin markiert ist,
wobei der Schritt des Detektierens der Bindung des Polypeptids und des Autoantikörpers weiter die Schritte des Kontaktierens des Biotin-markierten Polypeptids mit einem festen Substrat, das mit Avidin oder Streptavidin markiert ist; des Aufbringens der Probe auf das feste Substrat, um eine Bindung zwischen dem Autoantikörper der Probe und dem an das feste Substrat gebundenen Polypeptid zu ermöglichen; und des Aufbringens eines Detektionsantikörpers, der spezifisch den an das Polypeptid gebundenen Autoantikörper detektiert, umfasst, wobei der Detektionsantikörper spezifisch ein menschliches IgG oder IgA erkennt und mit Chromophoren markiert ist, die ein Enzym, das alkalische Phosphatase, Biotin, beta-Galaktosidase oder Peroxidase einschließt, radioaktives Material oder ein Material, das Farbpartikel einschließlich kolloidaler Goldpartikel oder Farblatexpartikel einschließt, einschließen.

14. In vitro-Verwendung eines Peptids zur Detektion eines AQP5-Autoantikörpers, wobei das Peptid eine Aminosäuresequenz aufweist, die ausgewählt ist aus der Gruppe bestehend aus GCSMNPARSFGC (SEQ ID NO: 5), CMNPARSFGC (SEQ ID NO: 6), CWPSALPTC (SEQ ID NO: 2); und CNNNTTQGC (SEQ ID NO: 3).

## Revendications

1. Polypeptide ayant une séquence d'acides aminés choisie parmi le groupe constitué de GCSMNPARSFGC (SEQ ID N° 5), CMNPARSFGC (SEQ ID N° 6), CWPSALPTC (SEQ ID N° 2) ; et CNNNTTQGC (SEQ ID N° 3).

2. Polypeptide selon la revendication 1, dans lequel le polypeptide de SEQ ID N° 2, SEQ ID N° 3, SEQ ID N° 5 et SEQ ID N° 6 a une structure circulaire par la liaison formée entre les deux résidus cystéine contenus dans celui-ci.

3. Nucléotide codant le polypeptide de la revendication 1.

4. Vecteur comportant le nucléotide de la revendication 3.

5. Cellule comportant le vecteur de la revendication 4.

6. Utilisation in vitro d'une composition comportant le polypeptide selon les revendications 1 ou 2 pour détecter des autoanticorps AQP5.

7. Composition pharmaceutique comportant le polypeptide selon les revendications 1 ou 2 pour une utilisation dans le traitement du syndrome de Sjögren.

8. Procédé pour détecter des autoanticorps AQP5, comportant les étapes consistant à :
fournir le polypeptide selon les revendications 1 ou 2 ;
fournir un échantillon qui a été obtenu à partir d'un sujet nécessitant une détection d'autoanticorps AQP5 ; et
mettre l'échantillon en contact avec le polypeptide pour détecter une liaison d'autoanticorps AQP5 et du polypeptide et pour corréler les résultats de la détection avec la présence d'autoanticorps AQP5 dans l'échantillon.

9. Procédé selon la revendication 8, dans lequel le résultat de détection positif comparé à un témoin indique que l'échantillon est atteint du syndrome de Sjögren.

10. Procédé selon les revendications 8 ou 9, dans lequel l'échantillon est un sang total, un sérum, un plasma, une larme ou une salive.

11. Procédé selon l'une quelconque des revendications 8 à 10, dans lequel la détection est réalisée par un tri de cellules activé par fluorescence (FACS), une immunocoloration de cellules, une immunodiffusion radiale, une immunoélectrophorèse, une immunoprécipitation incluant une électrophorèse à courant inverse, un dosage radio-immunologique (RIA) ou un dosage d'immunoadsorption par enzyme liée (ELISA).

12. Procédé selon l'une quelconque des revendications 8 à 10,
dans lequel la liaison entre le polypeptide et l'auto-anticorps forme un complexe et la détection inclut une utilisation d'un anticorps de détection qui reconnaît spécifiquement l'anticorps dans le complexe, et
dans lequel l'anticorps de détection reconnaît spécifiquement l'IgG ou l'IgA humaine et est marqué avec des chromophores incluant une enzyme incluant une phosphatase alcaline, une biotine, une bêta-galactosidase ou une péroxydase ; une matière radioactive ; ou une matière incluant des particules colorées incluant des particules d'or colloïdales ou des particules de latex colorées.

13. Procédé selon l'une quelconque des revendications 8 à 10,
dans lequel le polypeptide est marqué avec une biotine,
dans lequel l'étape de détection de la liaison du polypeptide et de l'auto-anticorps comporte en outre les étapes consistant à mettre en contact le polypeptide marqué à la biotine avec un substrat solide marqué avec de l'avidine ou de la streptavidine ; appliquer l'échantillon sur le substrat solide pour permettre une liaison entre l'auto-anticorps de l'échantillon et le polypeptide attaché au substrat solide ; et appliquer un anticorps de détection qui détecte spécifiquement l'autoanticorps lié au polypeptide, dans lequel l'anticorps de détection reconnaît spécifiquement l'IgG ou l'IgA humaine et est marqué avec des chromophores incluant une enzyme incluant une phosphatase alcaline, une biotine, une bêta-galactosidase ou une péroxydase ; une matière radioactive ; ou une matière incluant des particules de couleur incluant des particules d'or colloïdales ou des particules de latex de couleur.

14. Utilisation in vitro d'un peptide pour détecter des autoanticorps AQP5, dans lequel le peptide a une séquence d'acides aminés choisie parmi le groupe constitué de GCSMNPARSFGC (SEQ ID N° 5), CMNPARSFGC (SEQ ID N° 6), CWPSALPTC (SEQ ID N° 2) ; et CNNNTTQGC (SEQ ID N° 3).
